# EUROPEAN PATENT APPLICATION

(11) **EP 2 103 304 A1**
(43) Date of publication of application: **23.09.2009**
(21) Application number: 07706804.7
(22) Date of filing: 16.01.2007
(51) Int. Cl.: A61K 31/164, A61K 35/74, A61P 25/28

(54) **COMPOSITION FOR IMPROVING BRAIN FUNCTION**

(71) Applicant: Mizkan Group Corporation, Handa-shi, Aichi 475-8585 (JP)
(72) Inventor: FUKAMI, Hiroyuki, Nagoya-shi Aichi 464-0817 (JP)
(74) Representative: Gille Hrabal Struck Neidlein Prop Roos
(86) International application number: PCT/JP2007/050477
(87) International publication number: WO 2008/087704

(57) **Abstract**

Provided is a substance which is a safe material such as a food component and an extract of a natural product and has a stronger effect of ameliorating a cerebral function (effect of ameliorating a metabolism of monoamines as an intracerebral neurotransmitter and ameliorating deterioration in memory and learning due to hypofunction of a cholinergic neuron). The present invention provides a composition for ameliorating a cerebral function, comprising an alkali-stable lipid from an acetic acid bacterium, N-acylsphinganine or N-2'-hydroxypalmitoyl-sphinganine, as an active ingredient.

## Description

### Technical Field

The present invention relates to a functional composition having an effect of ameliorating a cerebral function by oral ingestion. More specifically, the present invention relates to a composition for ameliorating a cerebral function comprising, as an active ingredient, an alkali-stable lipid extracted from an acetic acid bacterium, N-acylsphinganine or N-2'-hydroxypalmitoyl-sphinganine, and having a function of ameliorating memory and learning by ameliorating an abnormal neurotransmission.

### Background Art

In recent years, due to the aging population, neurological and psychiatric disorders have become a burden to the society increasingly. For example, learning and memory disorders which are caused by dementia occurring frequently in aging people have been a big problem. The number of dementia patients in Japan has exceeded one million already, and is estimated to increase, twenty years later, close to three millions.

Alzheimer's disease (hereinafter, may be referred to as AD), which is one of the dementias, is clarified to cause an abnormal neurotransmission in the brain. That is, in AD, degeneration or defect of the cholinergic neuron in the basal nucleus of Mynert in the forebrain is caused. In addition, with those phenomena, the following phenomena are confirmed: decrease in an activity of acetylcholine transferase as a synthetase of the acetylcholine; reduction in the concentrations of monoamines such as norepinephrine and serotonin; and reduction in the concentration of neurotrophic factors such as NGF (refer to Non-patent Document 1, for example).
Note that hypofunction of the cholinergic neuron is a phenomenon occurring not only in the brain of AD but also in the aged brain (refer to Non-patent Document 1, for example). In addition, various cases show the fact that disorder of monoamines relates strongly to various neurological and psychiatric disorders such as schizophrenia (refer to Non-patent Document 2, for example).

Regarding to a serious brain disorder such as the AD, various synthetic pharmaceuticals as agents for ameliorating cerebral functions are developed. For example, an acetylcholinesterase inhibitor expected to ameliorate the disorder by activating a cholinergic neuron is approved as a treatment agent for Alzheimer's disease at the present.
However; the acetylcholinesterase inhibitor is reported to cause side effects such as hepatic dysfunction, arrhythmia, and exacerbation of peptic ulcer and the like. Therefore, the synthetic pharmaceuticals have been problematic due to those bad effects.
In addition, the neurotrophic factors are reported to have an effect of ameliorating a cerebral function by direct administration thereof into the brain (refer to Non-patent Document 1, for example). However, there is another problem in that the neurotrophic factors cannot pass the blood-brain barrier in case of oral ingestion because the neurotrophic factors themselves are polymers, and hence, the effect cannot be expected.

On the other hand, there are proposed various food components and extracts of natural products, expected to prevent and ameliorate the neurological and psychiatric disorders by being ingested as daily meal without using the synthetic pharmaceuticals regularly.
For example, focusing attention on the fact that phospholipids such as phosphatidylcholine and phosphatidylserine, fatty acids such as docosahexanoic acid and arachidonic acid, ganglioside, and sphingomyelin are present as specific lipid components in the cerebral neuron and they have the same physiological action as the neurotrophic factors, there is proposed a composition for ameliorating a cerebral function containing phosphatidylcholine, phosphatidylserine, and docosahexaenoic acid as active ingredients (refer to Patent Document 1, for example).
However, it is reported that effects of ameliorating a cerebral function of the phosphatidylcholine, phosphatidylserine, and the like are not so strong that metabolismof monoamines is not ameliorated (refer to Non-patent Document 3 and Non-patent Document 4, for example).
From the background, it is desired to develop a safe substance having a strong effect of ameliorating a cerebral function.

Non-patent Document 1: Senile dementia and therapeutic agent, CMC Publishing CO., LTD., p. 37 to 44, p. 108 to 125, 1988
Non-patent Document 2: Japanisch-Deutsche Medizinisch Beriche, Vol. 33, p. 319 to 330, 1988
Non-patent Document 3: Medicina, Vol. 20, p. 2132 to 2133, 1983
Non-patent Document 4: Acta psychiatrica Scandinavica, Vol. 81, p. 265 to 270, 1990
Patent Document 1: JP 07-17855 A

### Disclosure of the Invention

### Problems to be solved by the Invention

An object of the present invention is to solve the above-mentioned conventional problems and provide a substance which is a safe material, but not synthetic pharmaceuticals, such as a food component or an extract of natural product, and has an additionally strong effect of ameliorating a cerebral function.

### Means for solving the Problems

The inventor of the present invention has extensively studied in view of the above problems. As a result, the inventor focused on acetic acid bacteria which have been used as vinegar-producing bacteria since ancient time and confirmed to be deemed highly safe.
Note that Caspian yogurt as a traditional food in Europe contains acetic acid bacteria and it is known that the acetic acid bacteria themselves have been eaten historically and been highly safe.
The inventor studied on a function of ameliorating a cerebral function of the acetic acid bacteria which are highly safe and have been eaten. As a result, the inventor found that an alkali-stable lipid from acetic acid bacteria, N-acylsphinganine or N-2'-hydroxypalmitoyl-sphinganine, have strong effects of ameliorating the hypofunction of a neurotransmission and ameliorating memory and learning, thereby completing the present invention.

In the brain hypofunction due to the dementias or aging, with deterioration in memory and learning, there are confirmed the hypofunctions of the neurotransmission such as decrease in the activity of acetylcholine transferase, reduction in the concentrations of monoamines such as norepinephrine and serotonin, and reduction in the concentration of the neurotrophic factors such as NGF.

As a method of examining a function of ameliorating the brain hypofunction using animals such as rats or mice, there are exemplified a method of examining directly amelioration of memory and learning by a behavioral test such as Morris water maze test and a method of examining amelioration of the neurotransmission by a biochemical test involving quantifying the content of an intracerebral neurotransmitter.
In addition, a large amount of sample to be administered is required for the test using an animal. Therefore, as a method of examining a function of ameliorating a cerebral function with a small amount of the sample, there is a method using a model cell line of a nerve cell or a primary culture cell line of a brain tissue. That is, by observing neurotrophic factor-like actions such as extension of a neurite and suppression of a nerve cell death, the amelioration of the neurotransmission can be examined.

The inventor of the present invention made full use of the above examination methods, to thereby confirm that oral ingestion of an alkali-stable lipid, which is extracted from an acetic acid bacterium, ameliorated the metabolism of monoamines as an intracerebral neurotransmitter and ameliorated deterioration in memory and learning due to hypofunction of the cholinergic neuron. Further, it was confirmed that N-acylsphinganine as a component in the alkali-stable lipid from the acetic acid bacterium and N-2'-hydroxypalmitoyl-sphinganine as a kind of the N-acylsphinganine ameliorated hypofunction of the neurotransmission by the neurotrophic factor-like actions.
Note that it was also confirmed that the alkali-stable lipid of the present invention does not contain phosphatidylcholine, phosphatidylserine, and docosahexaenoic acid, of which effects of ameliorating a cerebral function are reported. In addition, it was confirmed that the N-acylsphinganine and N-2'-hydroxypalmitoyl-sphinganine were different from the above substances of which the effects of ameliorating a cerebral function are reported.

That is, a first embodiment of the present invention relates to a composition for ameliorating a cerebral function, comprising an alkali-stable lipid from an acetic acid bacterium as an active ingredient.
A second embodiment of the present invention relates to a composition for ameliorating a cerebral function, comprising N-acylsphinganine as an active ingredient.
A third embodiment of the present invention relates to a composition for ameliorating a cerebral function, comprising N-2'-hydroxypalmitoyl-sphinganine as an active ingredient.
A fourth embodiment of the present invention relates to a composition for ameliorating a cerebral function according to any one of the first to third embodiments, which is a beverage or a food.
A fifth embodiment of the present invention relates to a composition for ameliorating a cerebral function according to the fourth embodiment, which is a vinegar.

### Effects of the Invention

According to the composition for ameliorating a cerebral function of the present invention, an alkali-stable lipid from an acetic acid bacterium, in particular, N-acylsphinganine or N-2'-hydroxypalmitoyl-sphinganine, is directly orally ingested, whereby a function of ameliorating deterioration in the memory and learning due to degeneration or defect of the cholinergic neuron, a function of ameliorating the hypofunction of the neurotransmission by ameliorating the metabolism of monoamines as an intracerebral neurotransmitter, and a function of ameliorating the hypofunction of the neurotransmission by a neurotrophic factor-like actions can be obtained.
Further, the composition for ameliorating a cerebral function of the present invention is a substance contained in an acetic acid bacterium which has been eaten and is excellent in safety.

### Brief Description of the Drawings

Fig. 1 is a view illustrating an outline of a preparation method for an alkali-stable lipid from an acetic acid bacterium of the present invention.
Fig. 2 is a schema illustrating an apparatus for Morris water maze test used in Example 2.
Fig. 3 is a graph illustrating latency to escape platform in Example 2.
Fig. 4 is a graph illustrating a retention time in a fourth quadrant in a probe trial of Example 2.
Fig. 5 is a graph illustrating number of times a rat crossed the platform location in the probe trial of Example 2.
Figs. 6 are graphs each illustrating a content of norepinephrine (NE) or a content of 5-hydroxyindole acetic acid (5-HIAA) as a metabolite of serotonin in a cerebral cortex measured in Example 3.
Figs. 7 are graphs each illustrating a content of norepinephrine (NE) or a content of 5-hydroxyindole acetic acid (5-HIAA) as a metabolite of serotonin in a hippocampus measured in Example 3.

### Best Mode for carrying out the Invention

Hereinafter, the present invention is described in detail.
The present invention according to the first embodiment provides a composition for ameliorating a cerebral function, comprising an alkali-stable lipid from an acetic acid bacterium as an active ingredient.
The present invention according to the second embodiment provides a composition for ameliorating a cerebral function, comprising N-acylsphinganine as an active ingredient.
The present invention according to the third embodiment provides a composition for ameliorating a cerebral function, comprising N-2'-hydroxypalmitoyl-sphinganine as an active ingredient.

The acetic acid bacterium used in the present invention is not particularly limited, and any one of acetic acid bacteria belonging to *Gluconacetobacter, Gluconobacter, Acetobacter, Asaia,* and *Asidomonas* can be used.

Specifically, examples of the acetic acid bacterium belonging to *Gluconacetobacterinclude Gluconacetobacter hansenii, Gluconacetobacter diazotrophicus, Gluconacetobacter* intermedius, *Gluconacetobacter sacchari, Gluconacetobacter xylinus, Gluconacetobacter europaeus,* and *Gluconacetobacter oboediens.*

In addition, examples of the acetic acid bacterium belonging to *Gluconobacter* include *Gluconobacter frateurii* and *Gluconobacter cerinus.*

Further, examples of the acetic acidbacteriumbelonging to *Acetobacter* include *Acetobacter tropicalis, Acetobacter indonesiensis, Acetobacter syzygii, Acetobacter cibinongensis, Acetobacter orientalis, Acetobacter pasteurianus, Acetobacter orleanensis, Acetobacter lovaniensis, Acetobacter aceti, and Acetobacter pomorum.*

Examples of the acetic acid bacterium belonging to *Asaia* include *Asaia bogorensis* and *Asaia siamensis.*

In addition, examples of the acetic acid bacterium belonging to *Asidomonas* include *Asidomonas methanolica.*

Further, as the acetic acid bacterium, in addition to the above-mentioned bacteria, an acetic acid bacterium used in a vinegar production or a fermented food such as yogurt, an acetic acid bacterium isolated from the nature, and a stock stain which is preserved in microbial culture collections and can be shared are appropriately used.

The alkali-stable lipid from an acetic acid bacterium can be prepared by subjecting lipids, which are extracted from the acetic acid bacterium using an organic solvent consisting of one kind or two or more kinds selected from ethanol, acetone, hexane, chloroform, methanol, ethyl acetate, and the like, to weak alkaline decomposition treatment to thereby remove phospholipids. In view of the fact that safety is required for use in food and a low polar solvent is suitable for efficient extraction, hexane and acetone are suitable for the extraction solvent.

In addition, N-acylsphinganine or N-2'-hydroxypalmitoyl-sphinganine can be prepared by subjecting the alkali-stable lipid from the acetic acid bacterium to silica gel chromatography, eluting the N-acylsphinganine or N-2'-hydroxypalmitoyl-sphinganine using an organic solvent formed of two or more kinds selected from hexane, chloroform, methanol, and the like, and fractionating the eluted solution.
The N-acylsphinganine and N-2'-hydroxypalmitoyl-sphinganine which are synthesized chemically may be used, because the safety of the N-acylsphinganine and N-2'-hydroxypalmitoyl-sphinganine is backed up by a fact that the acetic acid bacterium has been eaten. In the case of the chemical synthesis of N-acylsphinganine and N-2'-hydroxypalmitoyl-sphinganine, it is desirable that no by-product causing a side effect contaminates them.

The alkali-stable lipid from an acetic acid bacterium includes hopanoids, sphingolipids, amino lipids, and fatty acids. Those compounds are generally present in membrane lipids of acetic acid bacteria and include the following compounds as described in "RESEARCH BULLETIN OF OBIHIRO UNIVERSITY", Vol. 23, p. 917 to 925 (1978), and "Doctor thesis of Graduate School of Agricultural Sciences, Iwate University", written by Hidetsugu Gotou, p. 11 to 41 (2001), for examples.

That is, examples of the hopanoids (terpenoid compounds) include tetrahydroxybacteriohopane(C35-pentacyclic terpene alcohol), hopane-22-ol, and hop-22 (29)-ene.
In addition, examples of the sphingolipids include: N-acylsphinganine such as N-2'-hydroxypalmitoyl-sphinganine, N-palmitoyl-sphinganine, 0-1-glucuronyl-N-2'-hydroxypalmitoyl-sphinganine, N-cis-vaccenoyl-sphinganine, and 0-1-glucuronyl-N-palmitoyl-sphinganine; and free sphinganine.
Further, examples of the amino lipids include 3-(palmitoyl)-hydroxypalmitoyl-ornithyl-taurine as ornithyl tarurine lipid, 3-(palmitoyl)-hydroxypalmitoyl-ornithine as ornithine lipid, and 3-hydroxypalmitoyl-ornithine as lyso-ornithine lipid.
Examples of the fatty acid include cis-vaccenic acid.

N-acylsphinganine refers to a kind of the sphingolipid contained in the alkali-stable lipid. The N-acylsphinganine is a compound generally named as ceramide in which a fatty acid binds to sphinganine as a sphingoid base and is generally represented by the following chemical formula (1). Note that, in the formula (1), -CO-R represents an acyl group. In the formula (1), as the fatty acids substituting the acyl group, there are known hydroxy fatty acids or normal fatty acids, saturated fatty acids or unsaturated fatty acids, linear fatty acids or branched chain fatty acids, and fatty acids having 2 to 30 or less carbon atoms.
Note that, in the case of N-acylsphinganine derived from acetic acid bacteria, fatty acids each substituting an acyl group such as myristic acid, palmitic acid, stearic acid, palmitoleic acid, oleic acid, vaccenic acid, linoleic acid, 2-hydroxymyristic acid, and 2-hydroxy palmitic acid are confirmed. In particular, N-2'-hydroxypalmitoyl-sphinganine, N-palmitoyl-sphinganine, N-cis-vaccenoyl-sphinganine account for most of the content ratio of the N-acylsphinganine in the case of acetic acid bacteria.

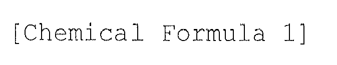

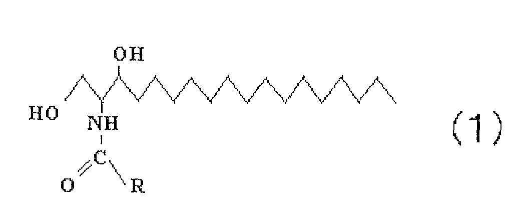

The content ratios of the above compounds are different from one another depending on the kind of the acetic acid bacteria. The compounds are present as main constituents of membrane lipids only in Gram-negative bacteria to which the acetic acid bacteria belong and are not present as lipid components in other organisms such as Gram-positive bacteria and eukaryote. Alternatively, the compounds are present in extremely trace amount.

The composition for ameliorating a cerebral function basically contains, as an active ingredient, an alkali-stable lipid from an acetic acid bacterium, which is prepared by extracting lipids from acetic acid bacteria using the above organic solvent and by subjecting the lipids to a weak alkaline decomposition treatment to thereby remove phospholipids. N-acylsphinganine and N-2'-hydroxypalmitoyl-sphinganine are obtained by purifying the alkali-stable lipid. However, the lipids without undergoing the weak alkaline decomposition treatment in the extraction step can be contained in the composition as it is, as required.

In addition, the alkali-stable lipid, N-acylsphinganine, or N-2'-hydroxypalmitoyl-sphinganine are present in the cell membrane of the acetic acid bacteria, and hence, the acetic acid bacteria without undergoing the extraction step, as it is, can be contained as an active ingredient. In this case, in view of the absorption in the body, cells of the acetic acid bacteria disrupted with a high-pressure homogenizer and the like are preferably contained.
Thus, even when the disrupted cells of the acetic acid bacterium containing the alkali-stable lipid, N-acylsphinganine and N-2'-hydroxypalmitoyl-sphinganine, or the lipids extracted from the acetic acid bacterium with an organic solvent or the like is contained as an active ingredient, the similar effect of ameliorating a cerebral function can be obtained.

As the dosage form of the composition for ameliorating a cerebral function of the present invention according to the first, second or third embodiment, a tablet, a capsule, a powder, or a liquid may be adopted.

Next, the present invention according to the fourth embodiment provides a composition for ameliorating a cerebral function according to any one of the first to third embodiments, which is a beverage or a food.
Here, examples of the form of the beverage or the food include: a confectionery such as a candy, gum, jerry, ice cream, or cookie; a staple food such as bread or rice; an alcohol beverage; a daily product such as a milk or yogurt; various kinds of beverages such as a vinegar beverage or sport beverage; and a seasoning such as a soy source, fermented soybean paste (miso), or vinegar.
Of those, the vinegar as described in the fifth embodiment is suitable, because it is expected to provide an excellent functional diet in combination with another healthcare function that the vinegar has.

In addition to the alkali-stable lipid from an acetic acid bacterium, N-acylsphinganine, or N-2'-hydroxypalmitoyl-sphinganine in the above-mentioned form, another composition for ameliorating a cerebral function derived from a natural product, an antioxidant derived from a natural product such as vegetables or fruits, and the like are blended, and thus, a synergistic effect in the amelioration of a cerebral function is expected.

In the present invention, the content ratio of the alkali-stable lipid from an acetic acid bacterium in the composition for ameliorating a cerebral function is 0.0001 to 5 wt% and preferably 0.001 to 1 wt%.
In addition, the content ratios of N-acylsphinganine and N-2'-hydroxypalmitoyl-sphinganine in the composition for ameliorating a cerebral function each are 0.00001 to 0.5 wt% and preferably 0.0001 to 0.1 wt%.

When the content of the alkali-stable lipid from an acetic acid bacterium in the composition for ameliorating a cerebral function is less than 0.0001 wt%, it is difficult to exhibit the effects of ameliorating an abnormal neurotransmission and ameliorating memory and learning. On the other hand, when the content exceeds 5 wt%, the composition is unsuitable for oral ingestion due to deterioration in the dispersion efficiency or the like upon production of the composition. Therefore, both cases are not preferred.
In addition, when the contents of the N-acylsphinganine and N-2'-hydroxypalmitoyl-sphinganine each in the composition for ameliorating a cerebral function are less than 0.00001 wt%, it is difficult to exhibit the effects of ameliorating an abnormal neurotransmission and ameliorating memory and learning. On the other hand, when the content exceeds 0.5 wt%, the composition is unsuitable for oral ingestion due to deterioration in the dispersion efficiency or the like upon production of the composition. Therefore, both cases are not preferred.

The dosage of the alkali-stable lipid from an acetic acid bacterium as the composition for ameliorating a cerebral function of the present invention is 0.001 mg to 100 g and preferably 0.1 mg to 10 g per adult per day. When the dosage is less than 0.1 mg per adult per day, the effects of ameliorating an abnormal neurotransmission and ameliorating memory and learning are less exhibited. On the other hand, when the dosage exceeds 10 g per adult per day, the composition may not be digested and absorbed.
In addition, the dosage of the N-acylsphinganine or N-2'-hydroxypalmitoyl-sphinganine is 0.0001 mg to 10 g and preferably 0.01 mg to 1 g per adult per day. When the dosage of the N-acylsphinganine or N-2'-hydroxypalmitoyl-sphinganine is less than 0.01 mg per adult per day, the effects of ameliorating an abnormal neurotransmission and ameliorating memory and learning are less exhibited. On the other hand, when the dosage exceeds 1 g per adult per day, the composition may not be digested and absorbed.

The composition for ameliorating a cerebral function of the present invention is produced by mixing uniformly with the alkali-stable lipid from an acetic acid bacterium, N-acylsphinganine, or N-2'-hydroxypalmitoyl-sphinganine as an active ingredient, various raw materials other than the active ingredient, and as required, a surfactant or the like to stabilize the composition. Examples of the surfactant include sorbitan fatty acid ester, glycerin fatty acid ester, propylene glycol fatty acid ester, sucrose fatty acid ester, and lecithin.

### Examples

Next, examples of the present invention are described in detail, but the scope of the present invention is not limited thereto.

### Example 1 (Preparation of alkali-stable lipid from acetic acid bacterium)

As an acetic acid bacterial strain, *Gluconacetobacter xylinus* NBRC15237 strain was used. The acetic acid bacterial strain is preserved in National Institute of Technology and Evaluation, Department of Biotechnology, Biological Resource Center (2-5-8, Kazusakamatari, Kisarazu-shi, Chiba-ken) and the acetic acid bacterium can be shared.

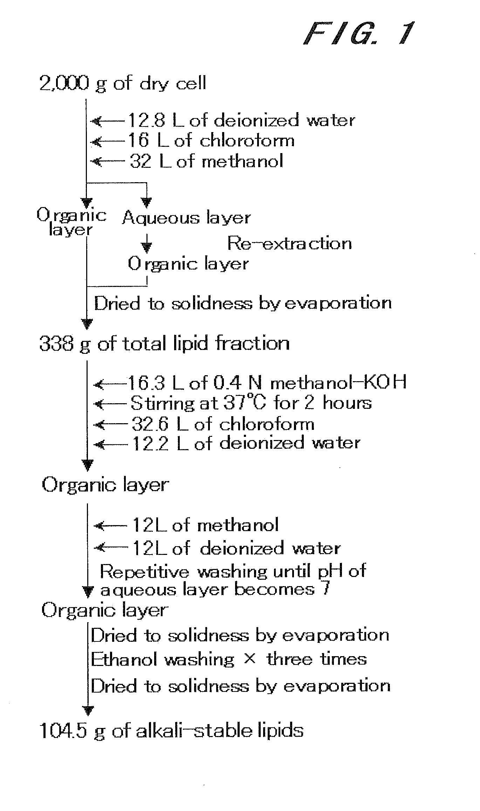

The alkali-stable lipid from the acetic acid bacterium was prepared by the following methods.
That is, 2,000 g of dry cells of the acetic acid bacterial strain was subjected to extraction with a chloroform-methanol solvent (chloroform:methanol:water=1:2:0.8), followed by separation into two layers. The lower layer was collected as a total lipid fraction. Next, the total lipid fraction was subjected to a weak alkaline decomposition treatment with 0.4 N NaOH to remove phospholipids, and the resultant was extracted again with a solvent according to the composition of Folch (chloroform:methanol:water =8:4:3). The resultant was dried to solidness by evaporation, whereby the alkali-stable lipid from the acetic acid bacterium was obtained. Fig. 1 shows the schema of the above extraction method.

The lipid components contained in the obtained alkali-stable lipid from the acetic acid bacterium were confirmed by thin layer chromatography using a silica gel plate. As a developing solvent in the thin layer chromatography, a solution having a ratio of chloroform to methanol of 96:4 was used for fatty acids, hopanoids, and sphingolipid, and a solution having a ratio of chloroform, methanol, to water of 65:16:2 was used for sphinganine and amino lipids.

As standard compounds, extracted products of an acetic acid bacterium confirmed to be hopanoids, N-acylsphinganine, or amino lipids (for example, refer to "RESEARCH BULLETIN OF OBIHIRO UNIVERSITY", Vol. 23, p. 917 to 925 (1978), and "Doctor thesis of Graduate School of Agricultural Sciences, I wate University", written by Hidetsugu Gotou, p. 11 to 41 (2001)) as well as a commercially available sphinganine (manufactured by SIGMA CORPORATION) were used. As a result of the thin layer chromatography, it was revealed that above standard compounds were present in the alkali-stable lipid.

In addition, the contents of the main components in the alkali-stable lipid were confirmed by high performance liquid chromatography. The alkali-stable lipid was reacted in the presence of anhydrous pyridine and benzoyl chloride at 70°C for 10 minutes, whereby benzoyl derivatives of hopanoids and a sphingolipid were purified from the resultant. The benzoyl derivatives were subjected to high performance liquid chromatography and were detected by an ultraviolet absorption at a wavelength of 230 nm.
The content of each compound present in the alkali-stable lipid was calculated from a standard curve obtained by using commercially available compounds or the standard compounds purified from the acetic acid bacterium of which the chemical structure had been confirmed. As amobilephase of the high performance chromatography, a solvent having a ratio of hexane to isopropanol of 100:1 was used and a flow rate was set to 1 ml/minute.

Table 1 below shows the thus analyzed main components in the alkali-stable lipid from the acetic acid bacterium. Note that the presence of the amino lipids was confirmed (the content ratio is unconfirmed), but the presence of phospholipids such as phosphatidylcholine, phosphatidylserine, and phosphatidyl glycerol was not confirmed.

**[Table 1]**

| Main component of alkali-stable lipid from acetic acid bacterium | Content ratio (weight/weight%) |
|---|---|
| Hopanoids | 11.511 |
| N-acylsphinganine | 5.446 |
| Sphinganine | 3.173 |

### Example 2 (Amelioration effect by administration of alkali-stable lipid from acetic acid bacterium in Morris water maze test using memory disorder model rat)

In the brain of AD patients and aging people, degeneration or defect of the cholinergic neuron is found in the basal nucleus of Mynert in the forebrain and the similar phenomenon can be also expressed by injecting ibotenic acid in the brain of a normal rat. Then, according to a conventional method, 5 µg of ibotenic acid as a neurotoxin were injected in the basal nucleus of Mynert in the forebrain of 7-week-old Crj:Wister male rats, whereby memory disorder model rats with defect in the cholinergic neuron were prepared.
The acetylcholine esterase inhibitor which is approved as a therapeutic agent for AD is reported to ameliorate the learning ability of the memory disorder model rat in a behavioral test by oral administration.
The alkali-stable lipid from the acetic acid bacterium prepared in Example 1 was orally administered to the memory disorder rats for 14 days from the day when ibotenic acid was injected, whereby the effect of ameliorating deterioration in the memory and learning due to defect in the cholinergic neuron was confirmed by a behavioral test, i.e. Morris water maze test.

### (1) Administration of alkali-stable lipid from acetic acid bacterium to memory disorder model rat

The rats were divided into the following five groups: an ibotenic acid-untreated group in which normal rats without memory disorder were used; an non-administration control group in which memory disorder model rats were used; a group in which the alkali-stable lipid from the acetic acid bacterium in low dose was administered (hereinafter referred to as low dose ASL group in some cases); a group in which the alkali-stable lipid from the acetic acid bacterium in high dose was administered (hereinafter referred to as high dose ASL group in some cases); and a positive control group in which the acetylcholine esterase inhibitor was administered (hereinafter referred to as acetylcholine esterase inhibitor-administrating group in some cases). Each group consisted 10 rats.

Note that rats in each group were allowed to ingest freely a solid feed of CRF-1 (manufactured by Oriental Yeast Co., Ltd.). In addition, in the groups in which the alkali-stable lipid from the acetic acid bacterium was administered, the alkali-stable lipid dispersed in 3% monomyristic acid solution was contained in the feed. The feed containing 165 mg of the alkaline-stable lipid per 1 kg of body weight per day and the feed containing 1,650 mg of the alkaline-stable lipid per 1 kg of body weight per day were orally administered in a low dose ASL group and in a high dose ASL group, respectively.
In the ibotenic acid-untreated group and the non-administration control group, only the 3% monomyristic acid solution was administered in the same volume as in the groups in which the alkali-stable lipid from the acetic acid bacterium was administered.
In the positive control group, 1 mg of 9-amino-1,2,3,4-tetrahydroacridine hydrochloride (manufactured by SIGMA CORPORATION) as an acetylcholine esterase inhibitor per 1 kg of rat weight per day, as a pharmaceutically effective amount, was orally administered. During all administration period for 14 days, there was no abnormality with respect to the weight and appearance of the rats in all groups.

### (2) Morris water maze test

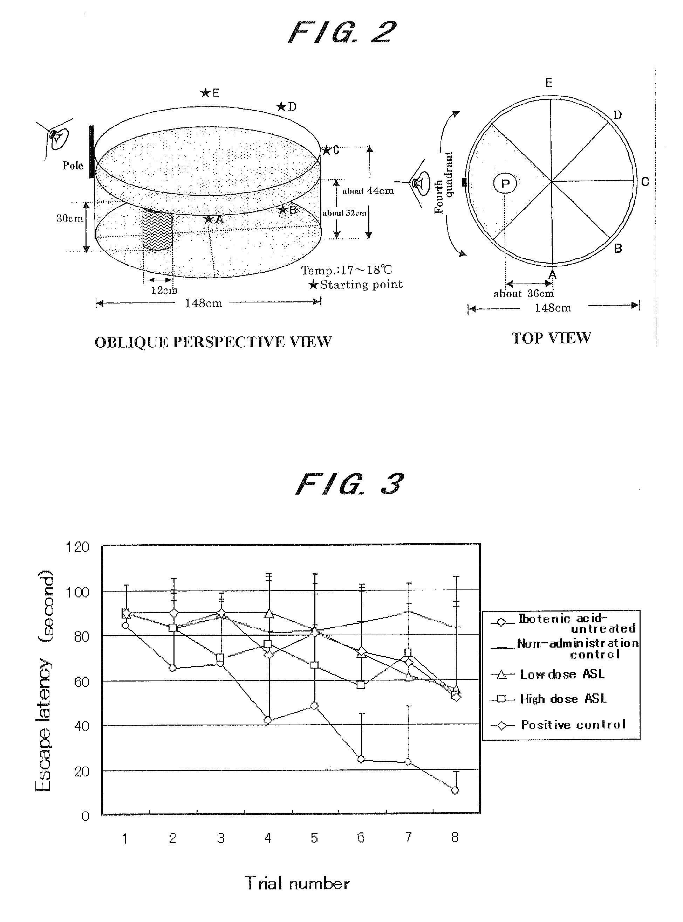

Fromday 10 to day 13 after the initiation of the administration, acquisition trials were performed for Morris water maze test, whereby the degree of learning was confirmed. Two trials were conducted for one rat in the morning and in the afternoon of the day and 8 trials were conducted in total of 4 days. Note that Fig. 2 illustrates a schema of a Morris water maze apparatus. There were used a transparent acrylic platform (diameter: about 12 cm, height: about 30 cm) which cannot be identified visually and a gray round pool (diameter: about 148 cm, height: about 44 cm) made of a vinyl chloride containing water up to the height of about 32 cm so as to hide the platform.
In addition, the pool was divided into four quadrants, a platform was provided in the center of the fourth quadrant (36 cm away from the center of the pool) and an electric bulb was provided around the pool as a spatial cue. The rat was put with its head toward the wall of the round pool from one of A to E points. The time (second) taken for the rat to reach the platform (escape latency) was measured (the measurement time was 90 seconds at the maximum) .
In the case where the rat reached the platform within a maximum measurement time of 90 seconds and stayed on the platform for 30 seconds, it was judged that the rat recognized the position of the platform and then the measurement was terminated. Note that the rat which could not reach the platform was rescued at the time of 90 seconds.
The place where the rats were put for 8 trials was C point, E point, A point, D point, B point, E point, C point, and A point in the stated order.

After the termination of the acquisition trials, probe trials were conducted on day 14 after the initiation of the administration.
That is, the platform was removed and how much time the rat stayed in the vicinity of the platform was observed, whereby the degrees of the spatial memory and acquisition of the place learning of the rat was confirmed. The head of a rat was directed to the wall of the round pool and put therein at C point. Then, the retention time (swimming time in the fourth quadrant: second) for which the rat stayed in the fourth quadrant (the quadrant where the platform was located upon the acquisition trial) and the number of crossings of the position where the platform was located (how many times a rat crossed the location where the platform was located in the fourth quadrant) were measured during observation times of 0 to 30 seconds, 30 to 60 seconds, and 60 to 90 seconds.

Figs. 3 to 5 show results of the acquisition trial and probe trial. Note that any result was represented by an average value of 10 rats for each group.

### (3) Influence of alkali-stable lipid on acquisition trial

Eight acquisition trials were conducted for the ibotenic acid-untreated group, non-administration control group, low dose ASL group, high dose ASL group, and positive control group. Fig. 3 illustrates the measurement results of the escape latency in the acquisition trial in each group. Note that the bars in Fig. 3 each represent standard deviation.
As a result, in the ibotenic acid-untreated group, with increase in the number of the trials, the escape latency was shortened because the learning effect was obtained. In addition, in the non-administration control group, the learning effect could not be obtained, and hence the escape latency was not shortened.
Meanwhile, the escape latency in low dose ASL group, high dose ASL group, and positive control group tended to be short as compared to the non-administration control group. In particular, the escape latency in the high dose ASL group was shortened compared to that in the positive control group in the third, fifth, and sixth trials, and the learning effect in the high dose ASL group was confirmed to be higher than that in the positive control group.
Further, in the seventh trial, there was a significant difference between the non-administration control group and the low dose ASL group with a critical rate of p<0.05 in Steel's test. On the contrary, there was no significant difference between the non-administration control group and the positive control group with a critical rate of p<0.05. Therefore, the learning effect in the low dose ASL group was also confirmed to be higher than that in the positive control group.

### (4) Influence of administration of alkali-stable lipid on probe trial

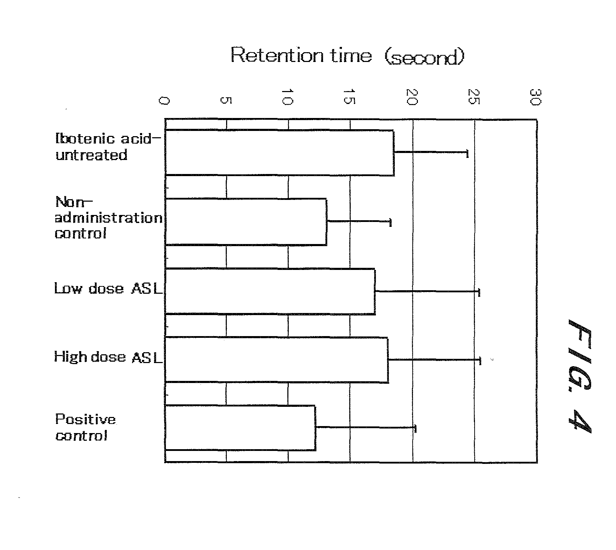

Fig. 4 illustrates the retention time in the fourth quadrant in the probe trials of the ibotenic acid-untreated group, non-administration control group, low dose ASL group, high dose ASL group, and positive control group. Note that the bars each represent a standard deviation.
As a result, in the non-administration control group, the retention time in the fourth quadrant was extended as compared to the ibotenic acid-untreated group. The retention time in the fourth quadrant of the positive control group did not tend to be long. On the contrary, each retention time of the low dose ASL group and high dose ASL group was extended to the level close to that of the ibotenic acid-untreated group, whereby the effect of ameliorating functions of acquiring spatial memory and place learning was confirmed to be higher than that of the positive control group.

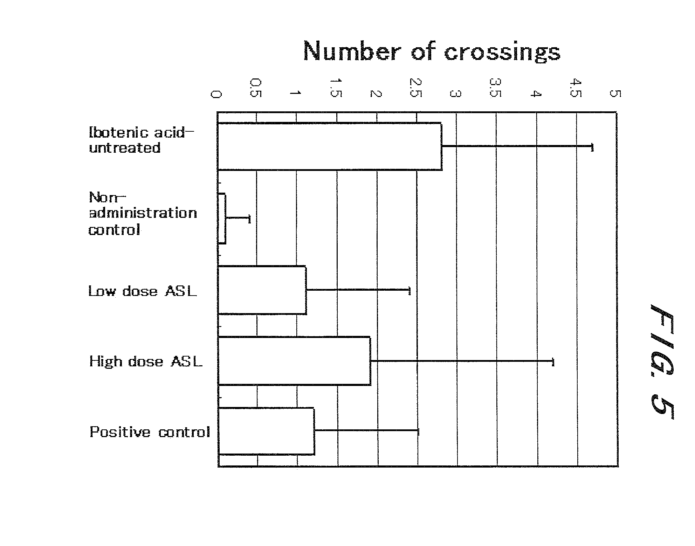

Further, Fig. 5 illustrates the number of crossings in the probe trials of the ibotenic acid-untreated group, non-administration control group, low dose ASL group, high dose ASL group, and positive control group. Note that the bars each represent a standard deviation.
As a result, the number of crossings in the non-administration control group was remarkably reduced in comparison with that in the ibotenic acid-untreated group. On the contrary, significant increase in the number of crossings was confirmed in the low dose ASL group, high dose ASL group, and positive control group. In particular, the number of crossings in the high dose ASL group was increased more than that of the positive control group, whereby the effect of ameliorating the functions of acquiring a spatial memory and place learning was confirmed to be equal to or higher than that of the positive control group.
Note that, there was a significant difference with a critical rate of p<0.05 in Steel' s test between the non-administration control group and each of three groups, i.e. the low dose ASL group, high dose ASL group, and positive control group.

### (5) Discussion

From the above results shown by the escape latency (Fig. 3) in the acquisition trials, the learning effect could be obtained at every trials in the ibotenic acid-untreated group without memory disorder and the escape latency was shortened every time the trial was repeated. In addition, the learning effect could not be obtained by the trials in the non-administration control group where the memory disorder due to the treatment with ibotenic acid was confirmed, and the escape latency was not shortened.
On the contrary, the learning effects in the low ASL group and high ASL group equal to or higher than that in the positive control group could be obtained. That is, the escape latency in the high dose ASL group was shortened more than that in the positive control group in the third, fifth, and sixth trials. In addition, the escape latency in the low dose ASL group was significantly shortened with respect to that in non-administration control group in the seventh trial, but there was no significant shortening in the positive control group.
From the foregoing, there was revealed an excellent effect of ameliorating the learning ability by administration of the alkali-stable lipid from the acetic acid bacterium.

Further, the same ameliorating effects were confirmed in the results of the probe trials (Fig. 4 and Fig. 5). That is, the retention time in the fourth quadrant was extended and the number of crossings was remarkably reduced in the non-administration control group where the ibotenic acid treatment was performed as compared to that in the ibotenic acid-untreated group. Thus, the functions of acquiring the spatial memory and the place learning were deteriorated in the non-administration control group.
On the contrary, the retention time in the fourth quadrant in the low dose ASL group and high dose ASL group was extended to the level close to that of the rat without the memory disorder. There was no ameliorating tendency in the retention time in the fourth quadrant observed in the positive control group. Further, significant increase in the number of crossings in the low dose ASL group and high dose ASL group was confirmed. The number of the crossings in the high dose ASL administration group was increased more than that in the positive control group.
From the foregoing, excellent effect of ameliorating the functions of acquiring the spatial memory and the place learning was confirmed by administration of the alkali-stable lipid from the acetic acid bacterium.

As described above, from the results of the acquisition trials and probe trials, it was confirmed behaviorally that, by the oral administration of the alkali-stable lipid from the acetic acid bacterium, excellent effect of ameliorating learning and memory disorder due to defect in the cholinergic neuron in the basal nucleus of Mynert in the forebrain found in the brain of AD patients or aging people could be exhibited.

Example 3 (effect of ameliorating intracerebral monoamines content by administering alkali-stable lipid from acetic acid bacterium to memory disorder model rat)
With the defect in the cholinergic neuron in the basal nucleus of Mynert in the forebrain found in the brain of AD patients or aging people, the concentrations of the monoamines such as norepinephrine and serotonin reduce in the cerebral cortex and hippocampus. The relationship between the abnormal metabolism of the monoamines and various neurological and psychiatric disorders is also confirmed.

Then, the increase and decrease in the concentrations of those intracerebral substances in rats of each group in Example 2 were measured.
That is, the brains of the memory disorder model rats prepared in Example 2 were extirpated and the contents of the monoamines in the cerebral cortex and hippocampus were measured.

### (1) Method of measuring monoamine content in the brain of memory disorder model rat

On day 14 after administration, 5 rats each in 5 groups of the ibotenic acid-untreated group, non-administration control group, low dose ASL group, high dose ASL group, and positive control group in Example 2 were decapitated with a dacapitation machine and the brains were taken out. The brains were put in an ice-cold physiological saline (Otsuka Pharmaceutical Co., Ltd.). Next, the brains were taken out and the cerebral cortex and hippocampus were then taken from the divided brains and the contents of the monoamines and a metabolite thereof were measured. The measurement method is shown in (a) to (c) below.

### (a) HPLC-ECD systems

Pump system EP-300 (manufactured by Eicom Corporation) Degasser DG-300 (manufactured by Eicom Corporation) Column thermostat ATC-300 (manufactured by Eicom Corporation) Electrochemical detector ECD-300 (manufactured by Eicom Corporation)
Graphite electrode WE-3G (manufactured by Eicom Corporation) Gasket GS-25 (manufactured by Eicom Corporation)
Reference electrode RE-100 (manufactured by Eicom Corporation)
Autosampler 231XL (manufactured by M&S Instruments Inc.)
Syringe pump 402 (manufactured by M&S Instruments Inc.) Condenser 832 (manufactured by M&S Instruments Inc.)
Data processing apparatus EPC-300 (manufactured by Eicom Corporation)
Homogenizer PRO 260 (manufactured by PRO Scientific Inc.)
Refrigerated centrifuge 5417R (manufactured by Eppendorf Yatron Co., Ltd.)

### (b) Pretreatment of sample

0.2 N hyperchloride solution was added to a brain sample in a container, and thereafter, an internal standard solution was added thereto, followed by homogenizing with a homogenizer. After being ice-cooled for 60 minutes or more, the resultant was centrifuged with a refrigerated centrifuge (about 4°C, 13,800 rpm, 5 minutes), and the supernatant was used as a measurement sample.

### (c) HPLC conditions

Separation column: Eicompak SC-50DS (3.0Ø×150 mm) (manufactured by Eicom Corporation)
Precolumn: PC-04 (filled with AC-ODS agent previously) (manufactured by Eicom Corporation)
Mobilephase: a mixture containing 0.1mol/L acetic acid-citric acid buffer (pH 3.5), methanol, 100 mg/ml sodium 1-octane sulfonate solution, and 5 mg/ml EDTA·2·Na solution at a ratio of 830:170:1.85:1
Flow rate: 0.5 ml/min
Column temperature: 25°C
Condensor temperature: 4°C
Injection volume: 20 µl
Applied voltage: +750 mV

### (2) Influence of administration of alkali-stable lipid on monoamine content in cerebral cortex and hippocampus

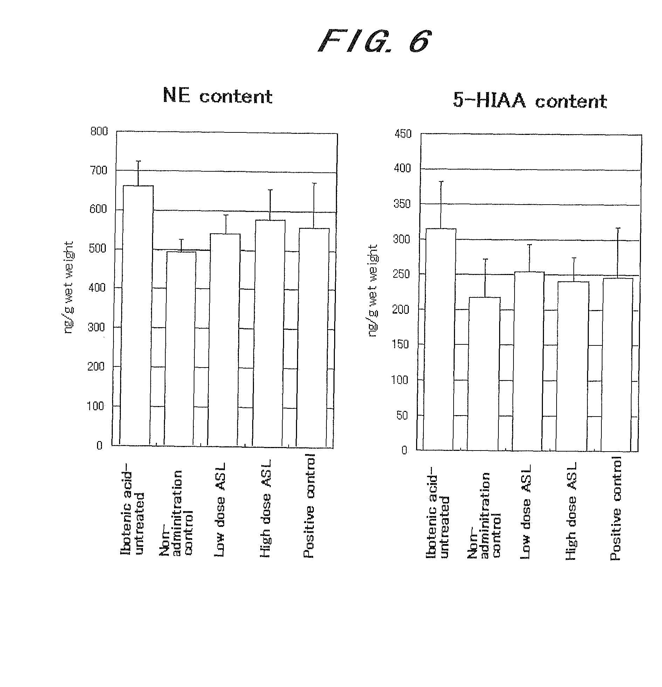

Fig. 6 illustrated the content of norepinephrine (NE) and the content of 5-hydroxyindole acetic acid (5-HIAA) as a metabolite of serotonin measured in the cerebral cortex of the ibotenic acid-untreated group, non-administration control group, low dose ASL group, high dose ASL group, and positive control group according to the above measurement method. The bars in Figs. 6 each represent a standard deviation.
As a result, increasing tendencies of the NE content and 5-HIAA content were shown in the low dose ASL group, high dose ASL group, and positive control group as compared to the non-administration control group.
Between the ibotenic acid-untreated group and non-administration control group, there was a significant difference in the NE content with a critical rate p<0.01 and in the 5-HIAA content with a critical rate p<0.01 in Student's t-test. On the contrary, there was no significant difference between the ibotenic acid-untreated group and the low dose ASL group, high dose ASL group, and positive control group with a critical rate p<0.05, whereby both the NE content and the 5-HIAA content of the low dose ASL group, high dose ASL group, and positive control group were confirmed to be recovered to the level close to those of the ibotenicacid-untreated group. Further, the NE content of the high dose ASL group was increased more than that of the positive control group, whereby the ameliorating effect in the high dose ASL group was confirmed to be equal to or higher than that of the positive control group.

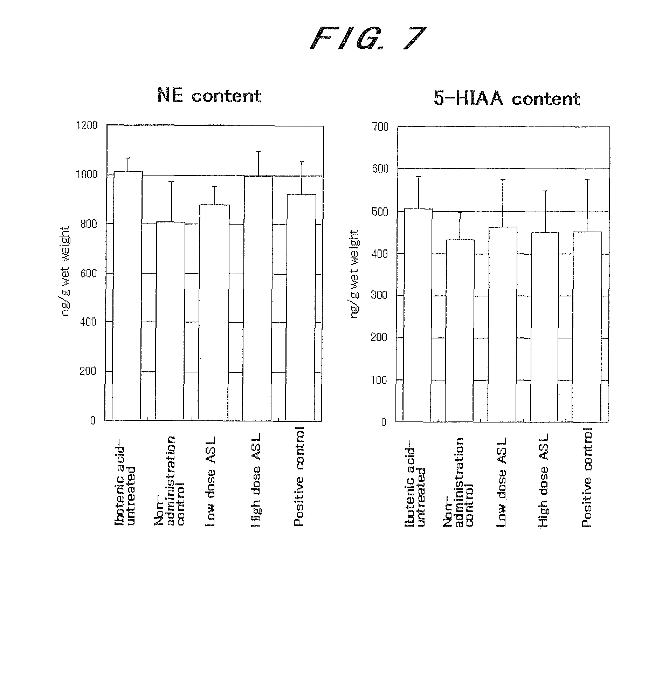

Similarly, Figs. 7 illustrate the measurement results of the NE content and 5-HIAA content in the hippocampus of the ibotenic acid-untreated group, non-administration control group, low dose ASL group, high dose ASL group, and positive control group. The bars in Figs. 7 each represent a standard deviation.
As a result, increasing tendencies of the NE content and 5-HIAA content were shown in the low dose ASL group, high dose ASL group, and positive control group as compared to the non-administration control group.
Between the ibotenic acid-untreated group and non-administration control group, there was a significant difference in the NE content with a critical rate p<0.05 in Student's t-test. On the contrary, there was no significant difference between the ibotenic acid-untreated group and the low dose ASL group, high dose ASL group, and positive control group with a critical rate p<0.05, whereby the NE content of the low dose ASL group, high dose ASL group, and positive control group was confirmed to be recovered to the level close to that of the ibotenic acid-untreated group. Further, the NE content of the high dose ASL group was increased more than that of the positive control group, whereby the ameliorating effect in the high dose ASL group was confirmed to be equal to or higher than that of the positive control group.
Note that the average value of 5 rats was shown in any of the results.

### (3) Discussion

From the above results, in the cerebral cortex, the NE content and 5-HIAA content of the non-administration group with memory disorder were significantly decreased in comparison with the ibotenic acid-untreated group without memory disorder. On the contrary, there were increasing or ameliorating tendencies in both the NE contents and 5-HIAA contents of both the low dose ASL group and high dose ASL group in comparison with those of the non-administration group. In addition, in the hippocampus, there were increasing or ameliorating tendencies in both the NE contents and 5-HIAA contents of both the low dose ASL group and high dose ASL group in comparison with those of the non-administration group with memory disorder. In the high dose ASL group, the NE contents in the cerebral cortex and hippocampus were increased more than those of the positive control group.

As described above, it was confirmed that the administration of the alkali-stable lipid from the acetic acid bacterium ameliorated the metabolism of the monoamines. In addition, from the effect of ameliorating the metabolism of the monoamines by oral administration of the alkali-stable lipid from the acetic acid bacterium, the oral administration of the alkali-stable lipid is expected to ameliorate not only the memory and learning in the brain of AD patients and aging people but also various kinds of neurological and psychiatric disorders in which the abnormal neurotransmission is involved.

### Example 4 (Fractionation of alkali-stable lipid from acetic acid bacterium)

The alkali-stable lipid prepared from the acetic acid bacterium in Example 1 was fractionated by chromatography depending on the strength of the polarity. Then, the chemical structure of the lipids was confirmed.
That is, 10 g of the alkali-stable lipid was loaded onto a silica gel column equilibrated with chloroform. The column was washed with a solvent prepared by mixing chloroform and acetic acid at a volume ratio of 100:1, and the elution was performed with a solvent having a ratio of chloroform to methanol of 97:3. The obtained fraction was dried to solidness, whereby about 0.5 g of a fraction 1 was obtained.
Subsequently, the fraction obtained by elution with solvents each having a ratio of chloroform to methanol of 96:4 and 95:5 was dried to solidness, whereby about 0.5 g of a fraction 2 was obtained. Further, the fraction obtained by eluting a remained lipid with a solvent having a ratio of chloroform to methanol of 2:1 was dried to solidness, whereby about 0.5 g of a fraction 3 was obtained.

The constituent lipids of the obtained fraction 1 were confirmed by thin layer chromatography using a silica gel plate. As the developing solvent in the thin layer chromatography, a solvent having a ratio of chloroform to methanol of 96:4 was used. As a result, the constituent lipids of the fraction 1 showed a single spot which had the same mobility as that of the purified product from the acetic acid bacterium identified to be the N-acylsphinganine used in Example 1. Further, the fraction 1 was decomposed with hydrous methanol-hydrochloride according to a known method and then separated into a sphingoid base and a fatty acid by thin layer chromatography, followed by purification.

To each sphingoid base and fatty acid, according to a known method, trimethyl silylation was performed and the chemical structure thereof was analyzed by gas chromatography and mass spectrometry. As a result, the fraction 1 was confirmed to contain N-2'-hydroxypalmitoyl-sphinganine, N-palmitoyl-sphinganine, and N-cis-vaccenoyl-sphinganine as N-acylsphinganine.
From the above results, the single spot detected by the thin layer chromatography of the fraction 1 was confirmed to be N-acylsphinganine.

Further, the constitute lipids in the fraction 2 and fraction 3 were confirmed by the thin layer chromatography. As the developing solvent in the thin layer chromatography, a solvent having a ratio of chloroform to methanol of 96:4 and a solvent having a ratio of chloroform, methanol, to water of 65:16:2 were used for the fraction 2 and fraction 3, respectively. As standards, the hopanoids and amino lipids used in Example 1, which were purified from the acetic acid bacterium and were confirmed in the chemical structures, and a commercially available sphinganine (manufactured by SIGMA CORPORATION) were used.

As a result of the thin layer chromatography, the fraction 2 showed a spot corresponding to that of the hopanoids as a standard and the fraction 3 showed a spot corresponding to that of sphinganine and the amino lipids as a standard.
From the above results, it was confirmed that the spot detected in the thin layer chromatography of the fraction 2 contained the hopanoids and the spot detected in the thin layer chromatography of the fraction 3 contained sphinganine and the amino lipids.

### Example 5 (Confirmation of neurite extension action)

Regarding three fractions purified in Example 4, i.e. the fraction 1 (containing N-acylsphinganine), the fraction 2 (containing the hopanoids), and the fraction 3 (containing sphinganine and the amino lipids), the absence or presence of an action of extending and differentiating the neurite as a neurotrophic factor-like action was confirmed using PC-12 cell as a neural model cell.

Note that, in the same manner as in Example 4, 10 g of the alkali-stable lipid was loaded into a silica gel column equilibrated with chloroform. The column was washed with a solvent prepared by mixing chloroform and acetic acid at a volume ratio of 100:1, and eluted with a solvent having a ratio of chloroform to methanol of 2:1, whereby a fraction containing all of the constituent lipids of the fraction 1, fraction 2, and fraction 3 was prepared. The fraction was used as an alkali-stable lipid fraction not containing a fatty acid. Then, the presence or the absence of the action of extending and differentiating the neurite was confirmed similarly.

PC-12 cell (RCB0009) purchased from RIKEN BIORESOURCE CENTER was used. The cell in a collagen V-coated dish was cultured in a medium containing Dulbecco's modified Eagle's medium (DMEM), containing 5% fetal bovine serum (FBS), 10% horse serum (HS), 100 µg/ml streptomycin, and 100 units/ml penicillin at 37°C and under 5% CO₂. The medium was changed once every two or three days, and subculture was performed once every 7 days with trypsin treatment.

The PC-12 cell being about 70% confluence was subjected to trypsin treatment, centrifugation, and resuspension. Then, the resultant was seeded to a collagen V-coated dish with 35 mm diameter at a concentration of 5×10⁴ cells/ml. The cell was cultured for several hours and the cell adhesion was formed on the dish. Then, the fraction 1, fraction 2, fraction 3, and the alkali-stable lipid dissolved in DMSO so as to have 100-fold concentrations of the addition concentrations were respectively added in a concentration of 1 %.

After that, the resultant was cultured at 37°C and under 5% CO₂ for 48 hours. Then, the morphologic feature and neurite of the cell were observed using a phase-contrast microscope. The cell having a neurite length equal to or longer than the cell diameter was defined as a differentiated cell. The total number of cells and the number of the differentiated cells in the viewing area were counted. Micrographs were photographed randomly so that one frame contained about 100 cells. Then, the average of 10 photographs in which the numbers were counted was calculated. A ratio of the number of the differentiated cells to the total number of the cells was defined as a differentiated cell ratio.

Table 2 shows the differentiated cell ratio of the PC-12 cell 48 hours after addition of the fraction 1, fraction 2, fraction 3, and alkali-stable lipid.
From Table 2, the differentiated cell ratio of the alkali-stable lipid increased more significantly compared to the case of the non-addition. In addition, when the fraction 1 was added in a concentration of 10 µg/ml, the differentiated cell ratio was confirmed to increase to larger extent compared to the case of non-addition.

From the above results, the fraction 1, i.e. the fraction containing N-acylsphinganine as a lipid component in the alkali-stable lipid, was confirmed to have the neurotrophic factor-like action. That is, it was revealed that ingestion of the N-acylsphinganine ameliorated the hypofunction of the neurotransmission due to neurodegenerative disease and aging.

**[Table 2]**

| Component | Addition concentration (µg/ml) | Differentiated cell ratio (%) of PC-12 cell 48 hours after addition |
|---|---|---|
| Non-addition | 0 | 4.20 |
| Fraction 1 | 10 | 11.26 |
| Fraction 2 | 10 | 3.11 |
| Fraction 3 | 10 | 4.78 |
| Alkali-stable lipid | 4 | 9.83 |
| | 20 | 15.18 |

### Example 6 (Confirmation of neurite extension action of N-2'-hydroxypalmitoyl-sphinganine)

The neurotrophic factor-like action of N-2'-hydroxypalmitoyl-sphinganine to the PC-12 cell was confirmed in the same way as in Example 5.
That is, the chemically synthesized N-2'-hydroxypalmitoyl-sphinganine (having purity of 99% or more, Matreya, LLC.), the fraction 1 (containing N-acylsphinganine) used in Example 5, and the alkali-stable lipid used in Example 5 dissolved in DMSO so as to have 100-fold concentrations of the addition concentrations were respectively added in a concentration of 1%. The other procedure was performed in the same manner as in Example 5.

Table 3 shows the differentiated cell ratio of the PC-12 cell 48 hours after the addition of each lipid.
From Table 3, by adding N-2'-hydroxypalmitoyl-sphinganine in a concentration of 5 to 25 µg/ml, similar to the case where the alkali-stable lipid or the fraction 1 was added, the differentiated cell ratio increased more significantly compared to the case of the non-addition.

From the above results, the neurotrophic factor-like action of the N-2'-hydroxypalmitoyl-sphinganine was confirmed. In addition, it was revealed that ingestion of the N-2'-hydroxypalmitoyl-sphinganine ameliorated the hypofunction of the neurotransmission due to neurodegenerative disease and aging.

**[Table 3]**

| Component | Addition concentration (µg/ml) | Differentiated cell ratio (%) of PC-12 cell 48 hours after addition |
|---|---|---|
| Non-addition | 0 | 9.85 |
| Fraction 1 | 5 | 19.49 |
| | 25 | 16.36 |
| N-2'-hydroxypalmitoyl-sphinganine | 5 | 19.49 |
| | 25 | 17.44 |
| Alkali-stable lipid | 20 | 22.08 |

### Example 7 (Preparation of tablet)

Cells in 10 kL of acetic acid-fermented broth were collected with a high speed centrifuge (8,000 rpm, 20 minutes), whereby 10 kg of wet cells were obtained. 10 kg of the obtained wet cells were washed with distilled water, and the resultant was then freeze-dried to solidness under reduced pressure with a large freeze-drying apparatus. Thus, 1.8 kg of dry cells were obtained.
1 kg of the obtained dry cells was loaded together with 10 L of ethanol into a Soxhlet extraction apparatus, followed by heat reflux for 20 hours. The obtained extracted solution was dried to solidness under reduced pressure and the obtained solid was dissolved in 5 L of acetone. The precipitant was removed by filtration and the filtrate was dried to solidness by evaporation with a rotary evaporator, whereby about 300 g of a lipid fraction containing a pale yellowish brown, the alkali-stable lipid of the acetic acid bacterium were obtained.
1 g (0.7 wt%) of the obtained lipids was added with 35 g (26.9 wt%) of crystalline cellulose, 67 g (51.5 wt%) of dry corn starch, 22 g (16.9 wt%) of lactose, 2 g (1.5 wt%) of calcium stearate, and 3 g (2.3 wt%) of polyvinyl pyrrolidone as a binder agent to produce a mixed powder. The obtained powder was filled in a gelatin hard capsule.
Thus, it is expected that the thus prepared tablet can be used effectively for oral ingestion as a composition for ameliorating cerebral function.

### Example 8 (Production of jelly)

Cells in 10 kL of acetic acid-fermented broth were collected with a high speed centrifuge (8,000 rpm, 20 minutes), whereby 10 kg of wet cells were obtained. 10 kg of the obtained wet cells were suspended in an equivalent amount of distilled water. 20 kg of the suspension of the acetic acid bacterium was subjected to cell disrupting treatment by being passed through a high pressure homogenizer (20,000 psi) three times. After that, the resultant was freeze-dried to solidness under reduced pressure with a large freeze-drying apparatus. Thus, 1.5 kg of dry cell powder was obtained.
8 g (0.8 wt%) of the obtained dry cell powder, 250 g (25 wt%) of sugar, 1.6 g (0.16 wt%) of carageenan, 0.8 g (0.08 wt%) of locust bean gum, and 0.8 g (0.08 wt%) of xanthan gum were uniformly mixed, whereby a powder mixture was obtained. 300 g of water were measured in a pot, 30 g (3.0 wt%) of brown sugar were dissolved therein. Further, 150 g (15 wt%) of a hydrogenated starch syrup were mixed therein, the powder mixture previously obtained was added, followed by further mixing under stirring. Those compounds were stirred uniformly and 258.8 g of the additional water were added thereto, followed by heating. Those compounds were dissolved by heating at the temperature of 85°C for 10 minutes. After that, the resultant was cooled with running water, whereby jelly food containing the cells of the acetic acid bacterium was obtained.
Thus, it is expected that the thus prepared jelly can be used effectively for oral ingestion as a composition for ameliorating cerebral function.

### Example 9 (Production of vinegar)

Cells in 10 kL of acetic acid-fermented broth were collected with a high speed centrifuge (8,000 rpm, 20 minutes), whereby 10 kg of wet cells were obtained. 10 kg of the obtained wet cells were suspended in 100 L of vinegar. The suspension of the acetic acid bacterium was subjected to cell disrupting treatment by being passed through a high pressure homogenizer (20,000 psi) three times. The obtained suspension was dispensed in a 500-ml volume bottle, and the bottle was sterilized by heating to 75°C. Thus, a vinegar containing the cells of the acetic acid bacterium was obtained.
Thus, it is expected that the thus prepared vinegar can be used effectively for oral ingestion as a composition for ameliorating cerebral function.

### Industrial Applicability

The present invention enables providing a substance having a strong effect of ameliorating a cerebral function, which is a safe material but not synthetic pharmaceuticals, such as a food component or an extract of a natural product. Accordingly, the composition for ameliorating a cerebral function according to the present invention is expected to have an effect of preventing and ameliorating neurological and psychiatric disorders by ingestion of the composition as a daily meal.

## Claims

1. A composition for ameliorating a cerebral function, comprising an alkali-stable lipid from an acetic acid bacterium as an active ingredient.

2. A composition for ameliorating a cerebral function, comprising N-acylsphinganine as an active ingredient.

3. A composition for ameliorating a cerebral function, comprising N-2'-hydroxypalmitoyl-sphinganine as an active ingredient.

4. A composition for ameliorating a cerebral function according to any one of claims 1 to 3, which is a beverage or a food.

5. A composition for ameliorating a cerebral function according to claim 4, which is a vinegar.
